# EUROPÄISCHE PATENTSCHRIFT

(11) **EP 2 844 314 B1**
(45) Veröffentlichungstag und Bekanntmachung des Hinweises auf die Patenterteilung: **21.02.2018**
(21) Anmeldenummer: 13720795.7
(22) Anmeldetag: 02.05.2013
(51) Int. Cl.: A61M 1/34, A61M 1/16

(54) **BILANZIERVORRICHTUNG DIALYSEGERÄT, EXTRAKORPORALER KREISLAUF UND VERFAHREN ZUR BILANZIERUNG VON FLÜSSIGKEITEN MIT EINER FLUSSMESSZELLE**
BALANCING DEVICE, DIALYSIS DEVICE, EXTRACORPOREAL CIRCUIT, AND METHOD FOR BALANCING LIQUIDS USING A FLOW MEASURING CELL
DISPOSITIF D'ÉTABLISSEMENT DE BILAN D'UN APPAREIL DE DIALYSE, CIRCULATION EXTRACORPORELLE ET PROCÉDÉ D'ÉTABLISSEMENT DE BILANS HYDRIQUES AU MOYEN D'UNE CELLULE DE MESURE DE DÉBIT

(30) Priorität: 04.05.2012 DE 102012009043; 04.05.2012 US 201261642631 P
(43) Veröffentlichungstag der Anmeldung: 11.03.2015
(73) Patentinhaber: Fresenius Medical Care Deutschland GmbH, 61352 Bad Homburg (DE)
(72) Erfinder: HEIDE, Alexander, 65817 Eppstein (DE); KONEGGER, Mario, 6020 Innsbruck (AT); NIKOLIC, Dejan, 65824 Schwalbach am Taunus (DE); PETERS, Arne, 61352 Bad Homburg (DE); WIKTOR, Christoph, 63571 Gelnhausen (DE)
(74) Vertreter: Dreyhsig, Jörg
(86) Internationale Anmeldenummer: PCT/EP2013/001294
(87) Internationale Veröffentlichungsnummer: WO 2013/164089

(56) Entgegenhaltungen:
- EP-A1- 0 898 975
- EP-A2- 0 904 789
- JP-A- 2000 084 071
- US-A- 5 024 756

## Beschreibung

### Technisches Gebiet

Die Erfindung betrifft eine Bilanziervorrichtung und ein Verfahren für die Bilanzierung einer Flüssigkeit, insbesondere von Dialysierflüssigkeit.

### Hintergrund

In einem Verfahren zur extrakorporalen Blutbehandlung wie der Hämofiltration, der Hämodiafiltration, der Hämodialyse, der Apherese und der Aquapherese wird dem Patienten während der Behandlung normalerweise Flüssigkeit einer genau vorab festgelegten Menge entzogen. Bei der Hämodialyse wird Blut in einem extrakorporalen Kreislauf mit einem Filter, der durch eine semipermeable Membran in zwei Abteile getrennt ist, zirkuliert. Das erste Abteil ist mit dem extrakorporalen Kreislauf verbunden, durch den das Blut fließt, und das zweite Abteil ist mit einem Dialysierflüssigkeitskreislauf verbunden durch den Dialysierflüssigkeit oder Dialysat, eine physiologische Lösung, fließt. Typischerweise beträgt die Menge an Dialysierflüssigkeit, die auf diese Weise durch den Filter geführt wird, 60 bis 2401 je Dialysebehandlung. Der Flüssigkeitsentzug erfolgt dabei durch ein Druckgefälle durch die semipermeable Membran von der Blutseite zur Dialysierflüssigkeitsseite. Die zu entziehende Flüssigkeitsmenge beträgt dabei typischerweise 2 bis 51.

Es ist von entscheidender Bedeutung, dass der Flüssigkeitsentzug mit großer Genauigkeit gemessen wird, denn schon ein geringfügig zu großer Flüssigkeitsentzug könnte gravierende Folgen für den Patienten haben.

In den bisher bekannten Geräten kommen zur Bilanzierung der Dialysierflüssigkeit entweder Bilanzkammern oder Flusssensoren zum Einsatz. Bilanzkammern stellen sicher, dass die Flüssigkeitsmenge in zwei Richtungen jeweils identisch ist, d.h. die zugeführte Flüssigkeitsmenge der abgeführten Flüssigkeitsmenge entspricht. Erreicht wird das durch eine volumenstarre Kammer, die durch eine flexible gas- und flüssigkeitsundurchlässige Membran in zwei Hälften geteilt ist, wobei jede Kammerhälfte mit je einem verschließbaren Zu- und Abflussventil versehen ist. Die Ventile werden dabei wechselweise geöffnet, sodass ein Zuflussventil und ein Abflussventil der jeweiligen anderen Kammerhälfte geöffnet bzw. geschlossen ist. Die durch das Zuflussventil einströmende Flüssigkeit deformiert die Membran derart, dass Sie die Flüssigkeit in der anderen Hälfte der Kammer verdrängt und die exakte selbe Flüssigkeitsmenge durch das geöffnete Abflussventil strömt.

Für das zusätzliche Entziehen von Flüssigkeit aus dem Patienten wird deshalb ein Flussweg mit Fördereinrichtung, die sog. Ultrafiltrationspumpe, parallel zur Bilanzkammer angeordnet. Die abzuziehende Flüssigkeit wird dabei über den parallelen Flussweg an der Bilanzkammer vorbei und durch die Ultrafiltrationspumpe gemessen. Bilanzkammern stellen hohe Anforderungen an die Fertigungstoleranz.

Alternativ können Flusssensoren wie Volumen- oder Massenstromsensoren eingesetzt werden, die die Zuflussmenge und die Abflussmenge separat erfassen. Aus der Differenz der gemessenen Flussmengen errechnet sich damit die entzogene Flüssigkeitsmenge. Die Verwendung von Volumen- oder Massenstromsensoren erfordert dabei eine hochgenaue Kalibrierung der Sensoren auf absolute Flüsse, wie beispielsweise in GB2003274 beschrieben, voraus. Diese Kalibrierung ist aufwändig und wird üblicherweise werksseitig vor Auslieferung des Dialysegeräts vorgenommen.

JP2000084071 offenbart ein Blutbehandlungsgerät mit einer Bilanziervorrichtung auf der Basis von Waagen. Dialysierflüssigkeit wird einem Dialyselösungskontainer entnommen und über eine Versorgungsleitung der Blutbehandlungseinheit zugeführt. Verbrauchte Flüssigkeit wird durch eine Abflussleitung geführt und in einem Abflusscontainer gesammelt. Zur Bilanzierung wird offenbart, dass das Ausgangsgewicht des Dialyselösungscontainer mit dem Gewicht des Abflusscontainers verglichen wird. Diese Differenz ist ein Maß für den gemessenen Wasserentzug und wird mit dem Zielwert des Wasserentzugs ("water removal target rate") verglichen. Die Korrekturpumpe wird angesteuert, so dass die zweite Differenz, d.h. zwischen dem gemessenen Wasserentzug und dem Zielwert des Wasserentzugs null wird. EP0904789 A2 offenbart ebenso relevanten Stand der Technik.

Es ist daher eine Aufgabe der Erfindung, zumindest eine der oben genannten Schwierigkeiten zu überwinden.

### Zusammenfassung

Diese Aufgabe wird durch eine Bilanziervorrichtung nach Anspruch 1 oder 2 und sowie durch ein Verfahren zur Bestimmung einer Flüssigkeitsbilanz gemäß Anspruch 13 gelöst. Vorteilhafte Ausführungsformen sind in den abhängigen Ansprüchen angegeben.

Der Differenzfluss kann als ein Differenzvolumen oder als Integral eines Differenzflusses ausgedrückt werden.

Nach einer vorteilhaften Ausführungsform beinhaltet die Einrichtung zur Einstellung der Flussmenge in dem weiteren Flussweg die Einrichtung zur Ermittlung der Flussmenge in dem weiteren Flussweg.

Dies stellt eine konstruktiv besonders einfache Ausführungsform dar.

Nach einer vorteilhaften Ausführungsform ist die Differenzflussmesseinheit als ein Differenzflusssensor zur unmittelbaren Messung des Differenzflusses zwischen dem Fluss im ersten Flussweg und dem Fluss im zweiten Flussweg ohne eine separate Messung des Flusses im ersten Flussweg oder eine separate Messung des Flusses im zweiten Flussweg ausgebildet.

Dies ist eine besonders einfache Ausführungsform der Differenzflussmesseinheit.

In einer weiteren Ausführungsform der Bilanziervorrichtung umfasst diese einen Differenzflusssensor mit einer erste Flusssmesszelle im ersten Flussweg und mit einer zur ersten Flussmesszelle gegenläufig durchströmten zweiten Flussmesszelle im zweiten Flussweg.

In dieser Ausführungsform können Flussmesszellen zum Einsatz kommen, die auf dem Gegenstromprinzip beruhen.

In einer weiteren Ausführungsform der Bilanziervorrichtung weist der weitere Flussweg ein absperrbares Ventil auf. Dadurch kann eine Absperrung des weiteren Flussweges, etwa zu Kalibrierungszwecken erfolgen.

In einer Ausführungsform ist die Bilanziervorrichtung oder ein Teil der Bilanziervorrichtung als Wegwerfartikel oder als Teil eines Wegwerfartikels ausgeführt, vorteilhaft als ein aus Kunststoff und für die einmalige Verwendung gefertigter Flusssensor.

Bei Wegwerfartikeln spielen eine Vielzahl an nicht oder nicht vollständig kontrollierbaren Faktoren eine Rolle, wie beispielsweise Lager- und Transportbedingungen und Alterung. Die beschriebene Bilanziervorrichtung kann hier auf relative Flüsse kalibriert werden. Eine Kalibrierung auf absolute Flüsse, wie sie im Stand der Technik bekannt ist, müsste bei einem Wegwerfartikel zur Erfüllung der Genauigkeitsanforderungen unmittelbar vor Einsatz, d.h. Behandlungsbeginn der Dialysebehandlung erfolgen. Nachteilig wäre hierbei, dass eine genau bekannte Flüssigkeitsmenge durch die Flusssensoren geleitet werden muss. Nachteilig wäre auch, dass diese Flüssigkeitsmenge hinreichend groß sein muss.

Insbesondere vorteilhaft kann ein derartiger Flusssensor, angewandt zur Bilanzierung von Dialyseflüssigkeit in mobilen oder portablen Dialysegeräten oder für Heimdialysesysteme zum Einsatz kommen. Vorteilhaft ist dabei dass die Herstellkosten für einen derartig gefertigten Flusssensor so gering gestaltet werden können, dass der Flusssensor die einmalige Verwendung je Behandlung ermöglicht. Dabei kann der Flusssensor vollständig oder teilweise in den extrakorporalen Kreislauf integriert werden, etwa in der Art dass der extrakorporale Blutkreislauf, einem Blutweg und einem Dialysierflüssigkeitsweg aufweist wobei der Dialysierflüssigkeitsweg die beschriebene Bilanziervorrichtung, angewandt zur Bilanzierung von Dialysierflüssigkeit in dem Dialysierflüssigkeitsweg enthält.

Bei der Anwendung in der Dialyse ist die Ausführung der Bilanziervorrichtung als Einwegartikel auch insoweit vorteilhaft, als dass der Flusssensor bei nur einmaliger Verwendung nicht oder nicht wesentlich mit im Dialysat enthaltenen Proteinen belegt wird. Zudem ist eine aufwendige Nachkalibrierung des Dialysegeräts in regelmäßigen Intervallen nicht erforderlich.

### Kurze Beschreibung der Zeichnungen

In Figur 1 ist schematisch ein Dialysegerät mit einer Bilanziervorrichtung im Einklang mit der erfindungsgemäßen Lehre dargestellt.
In den Figuren 2A und 2B ist jeweils eine Bilanziervorrichtung in einer nicht zur Erfindung gehörenden alternativen Ausführungsform dargestellt.
In den Figuren 3A und 3B sind weitere Bilanziervorrichtungen im Einklang mit der erfindungsgemäßen Lehre in einer weiteren vorteilhaften Ausführungsform dargestellt.
In den Figuren 4A und 4B ist jeweils eine schematische Darstellung einer Anordnung geeignet für die Kalibrierung einer Bilanziervorrichtung dargestellt.
In Figur 5 ist ein Flussdiagramm eines Verfahrens zur Flüssigkeitsbilanzierung dargestellt.
In Figur 6 ist ein weiteres Flussdiagramm eines Verfahrens zur Flüssigkeitsbilanzierung. dargestellt.
In Figur 7 ist das Prinzip der relativen Kalibrierung anhand verschiedener Flussraten dargestellt.

### Detaillierte Beschreibung der Zeichnungen

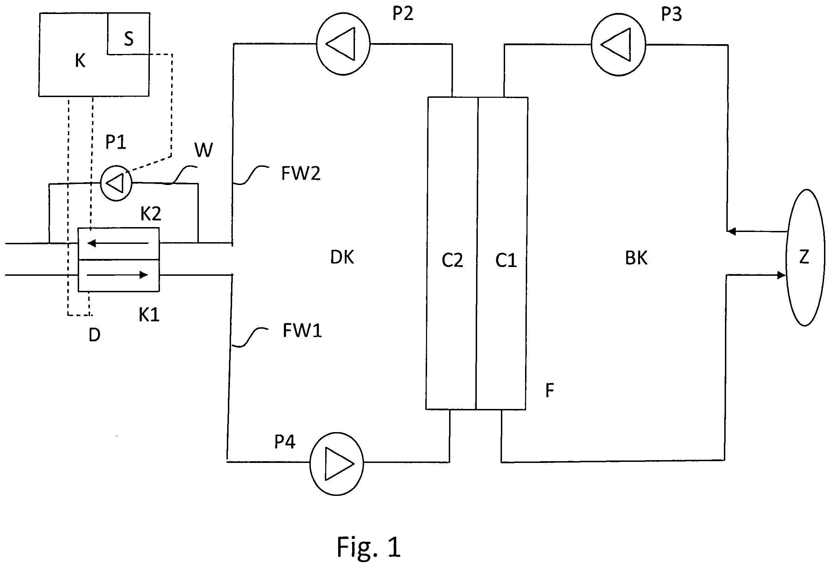

In Figur 1 ist schematisch ein Blutbehandlungsgerät 1 mit einem Bilanziersystem im Einklang mit der Lehre der vorliegenden Erfindung dargestellt. Das zu behandelnde Blut wird über einen Zugang Z dem Patienten entnommen und mit einer Pumpe P3 im Blutkreislauf BK durch eine Blutkammer des Filters F und über den Zugang Z an den Patienten zurückgegeben. Der Zugang Z verbindet den Blutkreislauf BK mit einem Blutgefäß des Patienten, das zur Blutentnahme und Rückgabe geeignet ist. Der Zugang Z kann zur Blutentnahme und zur Rückgabe des Blutes einen getrennten Ab- und Zufluss enthalten (,Double Needle'-Verfahren), oder Zu- und Abfluss können als ein Element ausgeführt sein (,Single Needle'-Verfahren).

Im Dialysator F trennt eine semipermeable Membran eine Dialysierflüssigkeitskammer C2 von einer Blutkammer C1. Über die semipermeable Membran findet ein Flüssigkeits- und Stoffaustausch von dem Blutkammer C1 in die Dialysierflüssigkeitskammer C2 statt. Durch die Dialysierflüssigkeitskammer C2 des Filters F wird Dialysierflüssigkeit mit einer Pumpe P2 stromab der Dialysierflüssigkeitskammer und mit einer Pumpe P4 stromauf der Dialysierflüssigkeitskammer transportiert. Der Zufluss zum Dialysator bildet so einen ersten Flussweg FW1 und der Abfluss vom Dialysator bildet einen zweiten Flussweg FW2. Die Flussrate in der Pumpe P2 ist um die Ultrafiltrationsrate höher als die Flussrate in der Pumpe P4. Durch den Unterschied der Flussraten in der Pumpe P2 und in der Pumpe P4 werden die Druckverhältnisse an der Membran im Dialysator F so eingestellt, dass in der Blutkammer C1 gegenüber der Dialysierflüssigkeitskammer C2 ein Überdruck herrscht. Dadurch erfolgt ein Transport von Flüssigkeit durch die Membran von der Blutkammer C1 in die Dialysierflüssigkeitskammer C2, diese Flüssigkeit wird Ultrafiltrat genannt. Durch ein Kontrollieren der Flussrate der Pumpe P2 und der Flussrate in der Pumpe P4 kann dabei die Ultrafiltratmenge oder Ultrafiltrationsrate eingestellt werden. Die Flussmesszellen K1 und K2 sind zu einem Differenzstromsensor D verbunden, wobei die Flussmesszelle K1 stromauf der Dialysierflüssigkeitskammer C2 und die Flussmesszelle K2 stromab der Dialysierflüssigkeitskammer C2 im Dialysierflüssigkeitskreislauf DK liegt.

Die Pumpe P1 bildet einen zur Flussmesszelle K2 parallelen Flüssigkeitsweg, in dem der Flüssigkeitstransport durch die Pumpe P1 kontrolliert wird.

Der Differenzstromsensor D ermittelt ein Messwertpaar bestehend aus einen separaten Messwert für jede Flussmesszelle K1, K2 der die Strömungsgeschwindigkeit der Flüssigkeit durch den Kanal in der jeweiligen Flussmesszelle anzeigt. Das Messwertpaar wird bevorzugt ein oder mehrmals pro Sekunde ermittelt und zu einem Kontroller K übertragen. Der Kontroller K ordnet jedem Messwertpaar ein Volumenstrompaar zu, wobei eine Abbildung von einem Messwert auf einen Volumenstrom verwendet werden kann, die auf einer zuvor durchgeführten Kalibrierung beruht. Alternativ könnte auch eine Abbildung auf einen Massenstrom erfolgen. Der Kontroller K leitet aus dem ermittelten Volumenstrompaar ein Steuersignal für die Pumpe P1 ab, etwa so, dass die Pumpe P1 derart betrieben wird, dass der Volumenstrom durch beide Flussmesszellen K1 und K2 des Differenzstromsensors zu jedem Zeitpunkt übereinstimmt. Beispielsweise bildet der Kontroller K die Differenz aus beiden Volumenströmen des Volumenstrompaares und verändert die Flussrate der Pumpe P1 durch Erhöhung oder Verringerung je nach Vorzeichen der Differenz in geeigneter Weise so, dass die Differenz zu null verschwindet. Ist der Fluss durch die Flussmesszelle K1 kleiner als der Fluss durch die Flussmesszelle K2, so ergibt sich für die Differenz der Messwerte von Flussmesszelle K2 und Flussmesszelle K1 ein positiver Wert. Der Kontroller K kann nun das Steuersignal für Pumpe P1 so verändern, dass sich die Flussrate durch Pumpe P1 erhöht und der Fluss durch Flussmesszelle K2 bei unverändertem Fluss durch Pumpe P2 soweit verringert bis sich der gleiche Fluss wie durch Flussmesszelle K1 einstellt. Die Flussrate durch die Pumpe P1 zeigt dann den Differenzfluss zwischen dem aus der Dialysierflüssigkeitskammer austretenden Flussweg und dem in die Dialysierflüssigkeitskammer eintretenden Flussweg an. Die Flussrate durch die Pumpe P1 ist dann ein Maß für die Menge des im Dialysator F entzogenen Ultrafiltrats.
In einer Ausführungsform werden die Flussrate durch die Pumpe P1 und die Flussrate durch die Pumpe P4 jeweils auf einen vorbestimmten Wert eingestellt, und die Flussrate durch die Pumpe P2 wird mittels einer Steuerleitung von dem Kontroller K zu der Pumpe P2 (nicht gezeigt) so eingeregelt, dass der im Differenzstromsensor D gemessene Differenzstrom eine vorbestimmte Bedingung erfüllt, etwa: zu null verschwindet.

Als Alternative können auch die Flussrate durch die Pumpe P1 und die Flussrate durch die Pumpe P2 jeweils auf einen vorbestimmten Wert eingestellt werden und die Flussrate durch die Pumpe P4 wird mittels einer (nicht gezeigten) Steuerleitung von dem Kontroller K so eingeregelt, dass der im Differenzstromsensor gemessene Differenzstrom eine bestimmte Bedingung erfüllt, beispielsweise zu null verschwindet.

Auch in diesen beiden Ausführungsformen ist die Flussrate durch die Pumpe P1 ein Maß für die Flüssigkeitsbilanz zwischen dem ersten Flussweg FW1 und dem zweiten Flussweg FW2, d.h. für die Menge des im Dialysator F entzogenen Ultrafiltrats.

In einer weiteren Ausgestaltung kann auf die Zuordnung des Messwertpaars zu einem Volumen- oder Massenstrom verzichtet werden, wenn die Differenz der Messwerte bei gleichem Volumenstrom durch beide Kanäle bekannt ist. In diesem Fall bildet der Kontroller K die Differenz aus beiden Messwerten und verändert die Flussrate der Pumpe P1 durch Erhöhung oder Verringerung der Differenz in geeigneter Weise solange bis die Differenz der vorbekannten Differenz bei gleichem Volumenstrom entspricht.

Der Differenzstromsensor D kann vorteilhaft nach dem Magentisch-Induktiven Prinzip funktionieren. Dabei weisen die beiden Flussmesszellen K1, K2, die im Gegenstrom durchströmt werden, einen rechteckigen Querschnitt auf und werden rechtwinkelig zu einem Magnetfeld angeordnet. Das Magnetfeld wird dabei von der Steuerung des Differenzstromsensors D eingestellt und ist so beschaffen, dass durch beide Flussmesszellen K1, K2 ein homogenes und gleich großes Feld vorherrscht. Dies wird beispielsweise dadurch erreicht, dass die Kanäle der Flussmesszellen K1, K2 übereinander zum Magnetfeld angeordnet sind. In jedem Kanal ist gegenüberliegend und im rechten Winkel zum Magnetfeld und zur Flussrichtung im jeweiligen Kanal eine Elektrode an derjenigen inneren Kanalwand angebracht, die sich entlang des Magnetfeldes erstreckt. Strömt Flüssigkeit durch den Kanal, so wird durch das Magnetfeld eine Ladungstrennung der in der Flüssigkeit vorliegenden Ionen bewirkt, sodass an den Elektroden eine elektrische Spannung vorliegt. Diese Spannung ist proportional zu der Strömungsgeschwindigkeit und abhängig von der Magnetfeldstärke. Ist das Magnetfeld in den beiden Flussmesszellen K1 und K2 gleich groß, so fällt bei der Bildung eines Differenzsignals aus beiden Kanälen die Magnetfeldstärkeabhängigkeit für das relative Differenzflusssignal vorteilhaft weg.

Mit anderen Worten: ein Verschwinden des Differenzsignals zeigt unabhängig von der absoluten Größe des Magnetfelds in den Flussmesszellen K1 und K2 an, dass der Fluss durch die Flussmesszelle K1 und der Fluss durch die Flussmesszelle K2 gleich groß sind.

Die Pumpe P1 ist bevorzugt aus der Gruppe der Verdrängerpumpen gewählt, bevorzugter eine Membran-, Schlauchrollen-, Kolben- oder Zahnradpumpe oder jede andere Pumpe die es erlaubt, die geförderte Flüssigkeitsmenge zu ermitteln. Beispielsweise kann mit der Schlauchrollenpumpe das geförderte Volumen durch das Pumpschlauchvolumen und dem Drehwinkel des Rotors der Schlauchrollenpumpe mit guter Genauigkeit mit bekannten Verfahren bestimmt werden. Auch für andere Pumpen aus der Gruppe der Verdrängerpumpen sind entsprechende Verfahren zur Bestimmung der geförderten Flüssigkeitsmenge aus dem Stand der Technik bekannt.

Vorteilhaft ist hierbei, dass die zu messende Flüssigkeitsmenge der Ultrafiltratmenge entspricht. Diese Menge liegt typischerweise bei 3-51 je Dialysebehandlung oder Tag, wohingegen die Menge an Dialysat, die durch den Flusssensor strömt, ein Vielfaches, typischerweise 60-2401 davon beträgt. Im Einklang mit der Lehre der vorliegenden Erfindung ist es daher vorteilhaft möglich, Messgeräte oder Messverfahren für den Differenzfluss einzusetzen, die eine wesentlich geringere Toleranz aufweisen müssen, als dies Messverfahren müssten, die die abfließende und die zufließende Menge an Dialysat getrennt erfassen, und erst anschließend eine Differenzbildung vornehmen.

Folgendes Rechenbeispiel verdeutlicht dies: Für eine Ultrafiltratmenge von 51 bedeutet ein Messfehler von 5% eine Menge von 250 ml Fehlbilanzierung. Würde ein derartiges Messverfahren mit einem Messfehler von 5 % im Dialysatkreislauf eingesetzt werden, in dem die abfließende und die zufließende Menge an Dialysat getrennt erfasst werden, und bei dem 601 Dialysat je Behandlung durch den Dialysator gefördert werden, so bedeutet ein 5%iger Messfehler eine Fehlbilanzierung von 31.

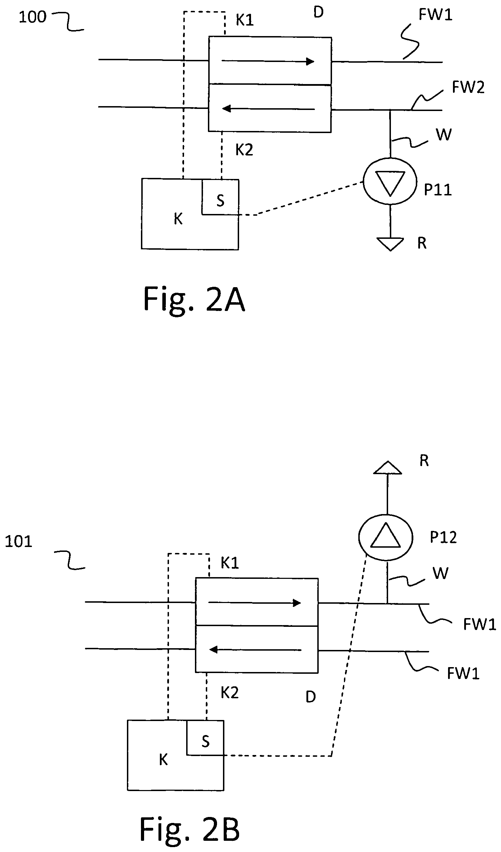

In den Abbildungen 2A und 2B ist jeweils eine Bilanziervorrichtung in einer nicht zur Erfindung gehörenden alternativen Ausführungsform zur Bestimmung einer Flüssigkeitsbilanz zwischen einer ersten Flussmenge in einem ersten Flussweg FW1 und einer zweiten Flussmenge in einem zweiten Flussweg FW2 dargestellt mit einer ersten Flussmesszelle K1 in dem ersten Flussweg FW1 und einer zweiten Flussmesszelle K2 in dem zweiten Flussweg FW2, wobei der eine der beiden Flusswege FW1, FW2 eine Verzweigung zur Abzweigung von Flüssigkeit in einen weiteren Flussweg W umfasst. Gleiche Bezugszeichen wie sie in Zusammenhang mit der Figur 1 verwendet und eingeführt wurden, zeigen in den Figuren 2A, und 2B gleiche oder entsprechende Elemente an. In den Bilanziervorrichtungen der Figur 2A zweigt der weitere Flussweg W von dem zweiten Flussweg FW2 ab, in den Figur 2B zweigt der weitere Flussweg W von dem ersten Flussweg FW1 ab. Die Bilanziervorrichtungen der Figuren 2A und 2B weisen jeweils eine Einrichtung zur Einstellung der Flussmenge in dem weiteren Flussweg W auf, und zwar jeweils eine Pumpe P11 beziehungsweise P12. Die Einrichtung zur Einstellung der Flussmenge in dem weiteren Flussweg ist derart ansteuerbar, dass die gemessene Flussmenge der ersten K1 und zweiten Flussmesszelle K2 eine vorbestimmte Bedingung erfüllt, vorzugsweise dass der Differenzflusses zwischen einem Fluss im ersten Flussweg FW1 und einem Fluss im zweiten Flussweg FW2 eine vorbestimmte Bedingung erfüllt, wie etwa dass der Differenzfluss null oder ungefähr null ist. Die Einrichtung zur Ermittlung der Flussmenge P11, P12 in dem weiteren Flussweg, mit anderen Worten die einstellbare Pumpe P11, oder die einstellbare Pumpe P12 dienen als Messeinheit für die Flüssigkeitsbilanz.

In der Anwendung als Flüssigkeitsbilanziersystem für die Dialyse ist der erste Flussweg FW1 ein Zufluss zu einer Dialysierflüssigkeitskammer eines Dialysators, der zweite Flussweg FW2 ist der Abfluss aus der Dialysierflüssigkeitskammer und die Flüssigkeitsbilanz ist ein Maß für die Menge des entzogenen Ultrafiltrats.
Die beiden Flussmesszellen können zu einem Differenzstromsensor D zusammengefasst werden, beispielsweise dem in GB 2003274 beschriebenen Differenzstromsensor. Bei dem in GB 2003274 beschriebenen Differenzstromsensor arbeiten die Flussmesszellen nach dem magnetisch-induktiven Prinzip bei dem beide Flussmesszellen einem gemeinsamen Magnetfeld ausgesetzt sind, so dass sich Variationen der Magnetfeldstärke auf beide Flussmesszellen gleichermaßen auswirken. Die durch die Flussmesszelle und senkrecht zum Magnetfeld hindurchströmende Flüssigkeit erfährt entsprechend der Lorentzkraft eine ladungstrennende Wirkung, so dass an den im Wesentlichen im rechten Winkel zum Magnetfeld und zur Strömungsrichtung angeordneten elektrischen Kontakten der Flussmesszelle eine Spannung gemessen werden kann. Die Flüssigkeit muss dazu elektrisch geladene Ionen oder dissoziierte Moleküle beinhalten, was typischerweise in der Dialysierflüssigkeit der Fall ist. Für andere, nicht nach dem magnetisch-induktiven Messverfahren arbeitende Flussmesszellen ist diese Anforderung nicht erforderlich.

Die Einrichtung geeignet zur Einstellung der Flussmenge in dem weiteren Flussweg kann als Pumpe, P11, P12 wie in Figur 2A und 2B gezeigt ausgeführt werden.

An dem Abfluss R kann dabei ein Behältnis, bevorzugt ein Beutel angeschlossen werden, der an einer Waage frei hängend oder aufliegend angebracht ist und der die durch den weiteren Flussweg transportierte Flussmenge sammelt. Ein solcher Beutel kann zugleich als Einrichtung zur Ermittlung der Flussmenge in dem weiteren Flussweg als auch als Behältnis zum Sammeln der Flussmenge dienen, wobei die Flussmenge gravimetrisch mit der Waage ermittelt wird. Damit wird es möglich, die Flussmenge auf diesem Weg zu erfassen. Vorteilhaft bei dieser Anordnung im Vergleich zu bestehenden Systemen zur Flüssigkeitsbilanzierung mit getrennten Beuteln für den Zu- und dem Ablauf ist, dass der beschriebene Beutel sehr klein sein kann und entsprechend an dem Gerät an einer vor mechanischen Einwirkungen geschützten Stelle angebracht werden kann. Damit wird auf eine vorteilhafte Weise verhindert, dass bei einem Wechsel des Dialysierflüssigkeitsbeutels während der Behandlung eine Waage für den Beutel und damit die Bilanzierung gestört wird. Eine Bilanzierung mit Waagen wird bislang bevorzugt in der Akutdialysetherapie verwendetet.

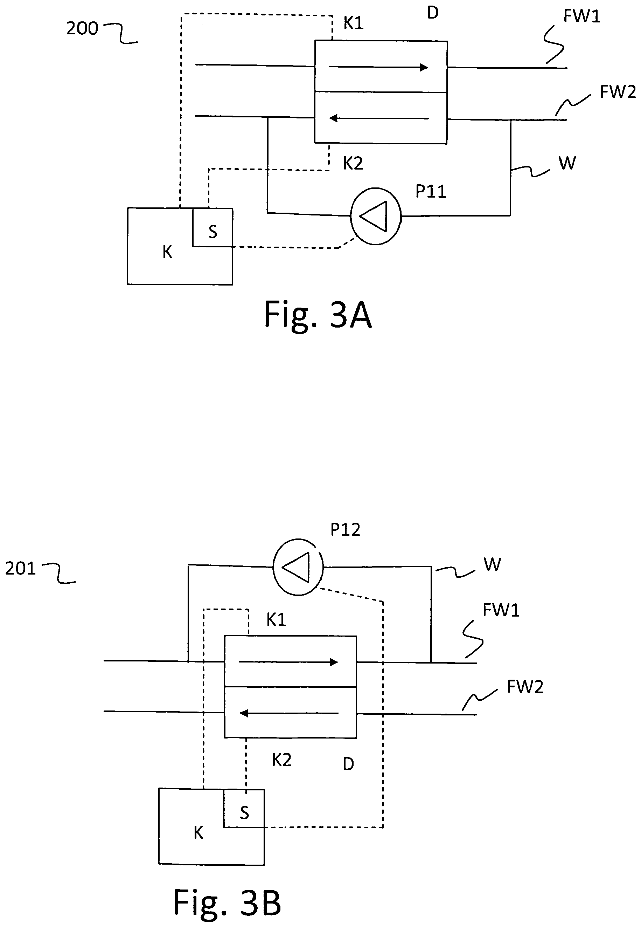

In den Figuren 3A und 3B sind weitere Bilanziersysteme im Einklang mit der Lehre der vorliegenden Erfindung in drei bevorzugten Ausführungsarten dargestellt. Gleiche Bezugszeichen wie in den Figuren 1 und 2A und 2B zeigen gleiche oder entsprechende Elemente an.

Den in den Figuren 3A und 3B dargestellten Bilanziersystemen ist gemeinsam, dass der weitere Flussweg W stromauf der ersten Flussmesszelle K1 (in dem Ausführungsbeispiel der Figur 3B) beziehungsweise stromab der zweiten Flussmesszelle K2 (in dem Ausführungsbeispiel der Figur 3A) wieder in den Flussweg der jeweiligen Flussmesszelle einmündet und so einen parallelen Flussweg zu dieser Flussmesszelle bildet. Die in den Figuren 3A und 3B dargestellten Bilanziersysteme weisen je einen Differenzstromsensor D mit gegenläufig durchströmten ersten und zweiten Flussmesszellen K1 und K2 auf. Durch die erste Flussmesszelle K1 fließt eine Flüssigkeit, in einer bevorzugten Ausführungsform Dialysat, mit einer ersten Flussrate. In der Ausführungsform der Figur 3A zweigt der weitere Flussweg W stromauf der zweiten Flussmesszelle K2 ab, führt über die Pumpe P11 und mündet stromab von Flussmesszelle K2 wieder ein und bildet so einen zu Flussmesszelle K2 parallelen Flussweg. In der in Figur 3B dargestellten Ausführungsform zweigt der weitere Flussweg W stromab der ersten Flussmesszelle K1 ab, führt über die Pumpe P12 und mündet stromauf der ersten Flussmesszelle K1 wieder in den ersten Flussweg FW1 ein und bildet so einen zu der ersten Flussmesszelle K1 parallelen Flüssigkeitsweg. So kann Flüssigkeit unter Kontrolle der Pumpe P11 an der zweiten Flussmesszelle K2 vorbeigeführt beziehungsweise unter Kontrolle der Pumpe P12 rückgeführt werden.

In der Anwendung als Flüssigkeitsbilanziersystem für die Dialyse können zwischen dem Differenzstromsensor und der Pumpe P11, beziehungsweise zwischen dem Differenzstromsensor und der Pumpe P12, die hier jeweils als Ultrafiltrationspumpe wirkt noch weitere (nicht gezeigte) Komponenten in dem Flüssigkeitsweg FW1, beziehungsweise in dem Flüssigkeitsweg FW2 enthalten sein, etwa eine Luftabscheidekammer oder eine Heizung für das Dialysat im Flüssigkeitsweg FW1.

Der Kontroller K umfasst einen Datenspeicher S, und ist mit der Pumpe P11 beziehungsweise Pumpe P12 so verbunden, dass der Kontroller K die Flussrate der Pumpe P11 beziehungsweise der Pumpe P12 einstellen kann. Die Verbindung kann auch dazu geeignet sein, die Flussrate zu bestimmen oder zu regeln.

Zusätzlich ist der Kontroller K mit dem Differenzstromsensor D über eine oder mehrere Leitungen verbunden. Der Differenzstromsensor D kann dabei eine Vorverarbeitung des Messsignals durchführen. Insbesondere kann der Differenzstromsensor je einen Messwert je Flussmesszelle K1, K2 getrennt oder als ein Messwertpaar, oder einen Messwert des Differenzflusses entsprechend an den Kontroller K übermitteln. Der Differenzstromsensor D ermittelt dabei bevorzugt in zeitlich diskreten Abständen aktuelle Messwerte, bevorzugter ein Mal pro Sekunde, noch bevorzugter mehrmals pro Sekunde. Der Kontroller ermittelt ein Steuersignal für die Pumpe P11 beziehungsweise für die Pumpe P12 basierend auf dem von dem Differenzstromsensor D erhaltenen Messwert und bildet so einen Regelkreis.

In einer beispielhaften Ausführung übermittelt der Differenzstromsensor D einen Messwert je Flussmesszelle K1, K2 also ein Messwertpaar zu einem bestimmten Zeitpunkt an den Kontroller K. Der Kontroller K ermittelt mit im Speicher S aus der Kalibrierung bekannten Parametern oder mit Hilfe einer Abbildung ein Steuersignal zur die Anpassung der Flussrate der Pumpe P11 beziehungsweise der Flussrate der Pumpe P12.

In dem in der Figur 3A dargestellten Ausführungsbeispiel wird Flüssigkeit durch den zur Flussmesszelle K2 parallelen Flussweg in derselben Flussrichtung wie in der Flussmesszelle K2 durch die Pumpe P11 transportiert. Diese Anordnung kann besonders dann eingesetzt werden, wenn der Fluss durch den zweiten Flussweg FW2 größer ist als durch den ersten Flussweg FW1, etwa wenn die Flussmesszelle K2 stromab von einem Dialysator angeordnet ist und zum Fluss durch den ersten Flussweg FW1 das Ultrafiltrat hinzugefügt wird.

In dem in Figur 3B dargestellten Ausführungsbeispiel wird Flüssigkeit durch den zur Flussmesszelle K1 parallelen weiteren Flussweg FW entgegen der Flussrichtung in der Flussmesszelle K1 durch die Pumpe P12 transportiert. Diese Anordnung kann besonders dann eingesetzt werden, wenn der Fluss durch den zweiten Flussweg FW2 kleiner ist als der Fluss durch den ersten Flussweg FW1. Das ist beispielsweise dann der Fall, wenn Flussmesszelle K1 stromauf vom Dialysator angeordnet ist und der Fluss durch den zweiten Flussweg durch das Ultrafiltrat größer ist.

In einer weiteren vorteilhaften Ausgestaltung kann im Flussweg der Flussmesszelle K2 ein Rückschlagventil angeordnet sein um Flüssigkeitstransport nur in der vorgesehenen Richtung stattfinden zu lassen.

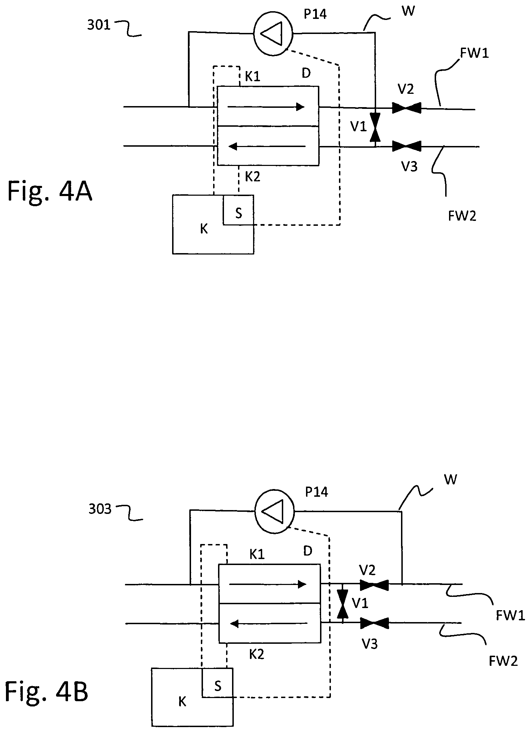

In den Figuren 4A und 4B ist schematisch jeweils eine Anordnung für die Durchführung der Kalibrierung dargestellt. Dabei ergänzen die in den Figuren 4A und 4B dargestellten Anordnungen jeweils die Anordnung der Figur 3B, um die Ventile V1, V2 und V3.

Die Kalibrierung kann vorteilhaft unmittelbar vor der Behandlung durchgeführt werden. Im Weiteren Vorteilhaft kann die Kalibrierung ohne vorbekannte Flussmenge durchgeführt werden. Für die Kalibrierung werden jeweils die Ventile V2 und V3 geschlossen und Ventil V1 wird geöffnet. Die Pumpe P14 wird in einen Zustand versetzt, sodass keine Flüssigkeit durch Pumpe P14 fließen kann.

Je nach eingesetzter Pumpe P14 kann auch ein zusätzliches, nicht in den Figuren 4A und 4B dargestelltes, verschließbares Ventil angeordnet vor oder nach der Pumpe P14 geschlossen werden.

In einer vorteilhaften Ausführungsform der Figur 4B ist das Ventil V3 derart angeordnet, dass durch Öffnen von Ventil V1 und gleichzeitigem Schließen der beiden Ventile V2 und V3 ein Flussweg ohne Abzweigung von Flussmesszelle K1 zu Flussmesszelle K2 gebildet wird. Vorteilhaft und entsprechend der Ausführung in Figur 4B kann Pumpe P14 für den Flüssigkeitstransport durch beide Flussmesszellen K1 und K2 während der Kalibrierung benutzt werden.

Wesentlich, und durch die in den Figuren 4A und 4B dargestellten Anordnungen sichergestellt, ist, dass beide Kanäle des Differenzstromsensors D einen durchgängigen Flussweg bilden, so dass dieselbe Flussmenge durch die beiden Flussmesszellen K1 und K2 fließt.

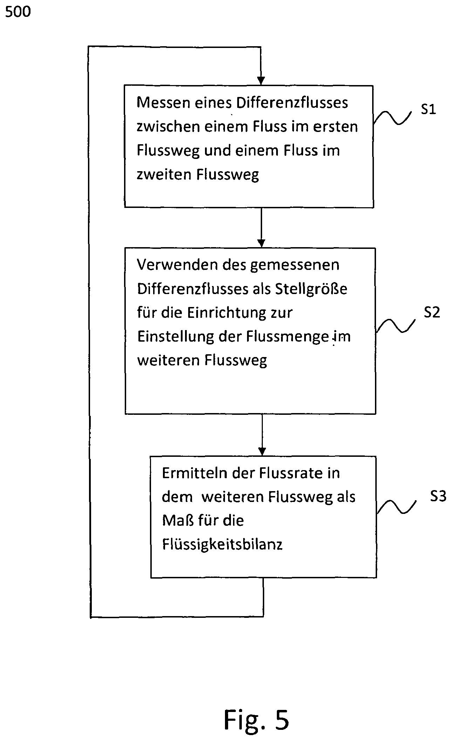

In der Figur 5 ist ein Verfahren zur Bestimmung einer Flüssigkeitsbilanz zwischen einer Flussmenge in einem ersten Flussweg und einer Flussmenge in einem zweiten Flussweg im Einklang mit der Lehre der vorliegenden Erfindung dargestellt. Das erfindungsgemäße Verfahren kann vorteilhaft mit einer der im Zusammenhang mit den Figuren 1, 2A, 2B, und 3B beschriebenen Bilanziervorrichtungen durchgeführt werden.

Das Verfahren umfasst die folgenden Schritte:
S1: Messen eines Differenzflusses zwischen einem Fluss im ersten Flussweg und einem Fluss im zweiten Flussweg
S2: Verwenden des gemessenen Differenzflusses als Stellgröße zur Erfüllung einer vorbestimmten Bedingung für die Einrichtung zur Einstellung der Flussmenge im weiteren Flussweg und
S3: Einstellen der Flussrate im weiteren Flussweg mit der Einrichtung, Ermitteln der Flussrate und Verwenden der Flussrate zum Ableiten eines Maßes für die Flüssigkeitsbilanz.

Die Flussmesszelle kann dabei ein Differenzstromsensor D wie in Figur 1, Figur 2A, 2B, 3A, 3B, 4A oder 4B dargestellt sein, es können aber auch andere Volumen- oder Massenstromsensoren verwendet werden, wobei sich das Differenzflusssignal erst in der Nachverarbeitung der Einzelflusssignale ergibt. Die Flussmesszellen können bereits den Messwert erfassen und vorverarbeiten und den so erhaltenen Wert an den Kontroller K übermitteln.

Der Kontroller K mit Speicher S empfängt die beiden Messwerte und ordnet mit den im Speicher aus der Kalibrierung bekannten Parametern Flussmengen zu. Die zugeordneten Flussmengen müssen dabei nicht unbedingt mit den tatsächlichen absoluten Flussmengen übereinstimmen, sondern müssen nur im Vergleich zueinander, d.h. relativ zueinander, stimmen. Die Zuordnung kann dabei über eine lineare oder eine andere geeignete Abbildung erfolgen, wobei die Parameter im Speicher S dann die Parameter der Abbildung sind. Die Parameter im Speicher S können aber auch abschnittsweise Funktionen oder Gruppen zugeordnet werden, so dass sich die Kalibrierung stückweise aus unterschiedlichen Abbildungen in bestimmten Flussratenbereichen über den gesamten Flussratenbereich zusammensetzt.

Die so erhaltenen Werte werden im Kontroller K verknüpft und mit der vorbestimmten Bedingung eine Stellgröße für die Einrichtung zur Einstellung der Flussmenge im weiteren Flussweg ermittelt. Vorteilhaft ist die Verknüpfung als Differenz- oder Summenbildung ausgebildet. Die Stellgröße wird vom Kontroller in Form eines Signals ausgegeben. Die Einrichtung zur Einstellung der Flussrate im weiteren Flussweg empfängt das Signal und verändert die Flussrate entsprechend. Das Signal kann dabei ein digitales oder analoges Signal sein. Die Einrichtung zur Einstellung der Flussrate kann dabei als einstellbare Pumpe ausgebildet sein.

Die vorbestimmte Bedingung ist beispielhaft erfüllt, wenn die Flussmenge durch den ersten und zweiten Flussweg gleich ist. Das erfindungsgemäße Verfahren und Vorrichtung funktionieren gleichermaßen bei beliebigen anderen vorbestimmten Bedingungen, wenn die Kalibrierung für beide Flussmengen durchgeführt wurde und die Flussmenge in einem der beiden Flusswege konstant gehalten werden kann ohne die weitere Einrichtung zur Einstellung der Flussmenge im weiteren Flussweg.

Die Schritte S1, S2 und S3 des beschriebenen Verfahrens werden zeitlich in dieser Reihenfolge wiederholt. Bevorzugt werden die Schritte S1, S2 und S3 zeitlich mindestens einmal pro Sekunde, bevorzugter mehrmals pro Sekunde wiederholt.

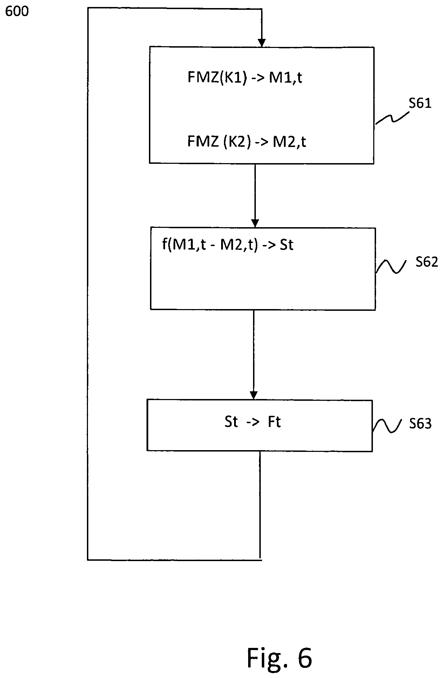

In Figur 6 ist das erfindungsgemäße Verfahren in einer weiteren vorteilhaften Ausgestaltung dargestellt. Das Verfahren funktioniert ähnlich wie das im Zusammenhang mit der Figur 5 beschriebene Verfahren, wobei in einem ersten Schritt S61 die Messwerte Mit und M2t einer ersten und zweiten Flussmesszelle FMZ1 und FMZ2, die jeweils die Flussmenge in einem ersten und in einem zweiten Flussweg K1 und K2 bestimmen und dem jeweiligen Messwert M1t, M2t einem entsprechenden Zeitpunkt t zuordnet. In Schritt S62 bildet der Kontroller K mit Speicher S die Differenz aus beiden Messwerten und leitet mit Hilfe der im Speicher S bekannten Parameter aus der Kalibrierung eine Stellgröße St für die Einrichtung zur Einstellung der Flussmenge im weiteren Flussweg ab. Der Kontroller K gibt in Schritt S63 die Stellgröße in Form eines analogen oder digitalen Signals an die Einrichtung zur Einstellung der Flussmenge Ft im weiteren Flussweg aus. Diese Verfahrensschritte werden zeitlich periodisch wiederholt, vorteilhaft einmal pro Sekunden, bevorzugt mehrmals pro Sekunde.

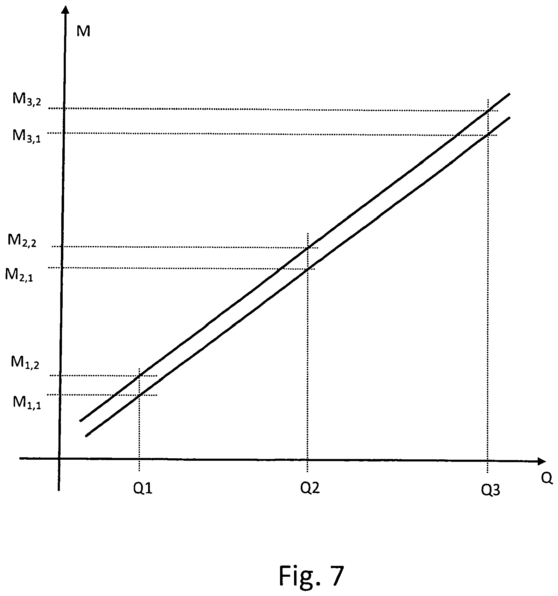

Ein Verfahren zur Kalibrierung einer in den Figuren 4A und 4B dargestellten Bilanziervorrichtung ist Figur 7 veranschaulicht.

Über eine in den Figuren 4A und 4B nicht dargestellte Pumpe wird Flüssigkeit durch die beiden Flussmesszellen K1 und K2 mit der gleichen vorbestimmten jedoch nicht notwendig bekannten Flussrate gepumpt. Der vom Differenzstromsensor D ermittelte Messwert je Flussmesszelle (K1, K2) wird an den Kontroller K übertragen. Dieser Zusammenhang ist beispielhaft in Figur 7 dargestellt. Dabei wird die vorbestimmte nicht näher bekannte Flussrate Q1 eingestellt, die vorteilhaft mit der Flussrate für das Dialysat während der Behandlung übereinstimmt. Der Differenzstromsensor D ermittelt einen Messwert M1,1 für die erste Flussmesszelle K1 und einen Messwert M1,2 für die zweite Flussmesszelle K2 und übermittelt das Messwertpaar an den Kontroller K. Die ermittelten Messwerte müssen dabei nicht den tatsächlichen absoluten Fluss durch die jeweilige Flussmesszelle anzeigen.

Der Kontroller K speichert beide Werte in der Speichereinheit S. In einer weiteren Ausgestaltung bildet der Kontroller beispielsweise die Differenz aus beiden Werten und speichert den Differenzwert in der Speichereinheit S. Die Erfindung ist nicht auf diese beiden Ausführungsbeispiele eingeschränkt und schließt weitere Ausführungen mit ein.

In einer vorteilhaften Weiterbildung wird die Kalibrierung mit mehreren verschiedenen Flussraten Q1, Q2 und Q3 wiederholt und die so ermittelten Werte oder Wertepaare (M2,1; M2,2 und M3,1; M3,2) separat im Speicher S gespeichert. Dabei werden vorteilhaft die Pumpen P2 und P4 in Figur 1 für den Dialysatkreislauf DK in Figur 1, typischerweise Peristaltikpumpen, benutzt und deren Flussrate mit einem aus dem Stand der Technik bekannten Verfahren ermittelt und an den Kontroller K mitgeteilt. Wichtig ist dabei die verschiedenen Flussrate derart zu wählen um den gesamten während der Behandlung benutzten Bereich zu erfassen, beispielsweise 100ml/min, 200ml/min, 500ml/min.
Es hat sich herausgestellt, dass der Zusammenhang zwischen Messwert und Flussrate im Wesentlichen linear ist. Vorteilhaft errechnet der Kontroller K mit den ermittelten Werten für eine während der Behandlung eingestellten Dialysatflussrate mit einer linearen Interpolation das zugehörige Messwertpaar.

Für das ordnungsgemäße Funktionieren des erfindungsgemäßen Bilanziersystems ist es nicht unbedingt erforderlich dass die beiden Kanäle gegenläufig durchströmt werden müssen.

Das beschriebene Verfahren und die beschriebenen Bilanziersysteme funktionieren ebenfalls mit anderen Flusssensoren, wie beispielsweise elektisch-induktiven, Coriolis- oder Flügelradsensoren und weitere aus dem Stand der Technik bekannte Flusssensoren. Insbesondere vorteilhaft werden Massenstromsensoren eingesetzt um Messfehler bedingt durch Luftblasen in der Flüssigkeit zu minimieren oder auszuschließen. Der Flusssensor kann dabei schon eine Vorverarbeitung der Messsignale von den Messeinheiten, beispielsweise Elektroden für Elektisch-Induktive Strömungssensoren, durchführen und ein digitales oder ratiometrisches Ausgangssignal an den Kontroller K übermitteln.

Das beschriebene Verfahren und die beschriebenen Bilanziersysteme entsprechend einem der Ausführungsformen aus den Figuren 2A, 2B, 3A, 3B, 4A und 4B können auch so ausgestaltet sein, dass die Flüsse durch Flussmesszelle K1 und Flussmesszelle K2 zeitlich versetzt durchlaufen werden und dass der Differenzfluss als ein Differenzvolumen, als ein Integral eines Differenzflusses, oder als Differenz eines Integrals des Flusses im ersten Flussweg und eines Integrals des Flusses im zweiten Flussweg ausgedrückt wird. Dabei wird beispielsweise zunächst Flüssigkeit durch Flussmesszelle K1 transportiert und Flussmesszelle K2 transportiert keine Flüssigkeit. Der Kontroller K zeichnet beispielhaft die Messwerte oder Messwertpaare die der Flusssensor während diesem Zeitraum übermittelt auf. Wird nun in einem zweiten Zeitraum der Flüssigkeitstransport durch Flussmesszelle K2 angehalten und Flüssigkeit durch Flussmesszelle K2 transportiert, kann der Kontroller den Messwert von Flussmesszelle K1 durch den zuvor aufgezeichneten Wert ersetzen und die Flussrate der Pumpe P1 mit dem so neu entstandenen Messwertpaar kontrollieren. Das Verfahren zur Bilanzierung mit dem beschriebenen Bilanziersystem kann auch so ausgestaltet sein dass die jeweilige Flussmesszelle anstatt keine Flüssigkeit zu transportieren Flüssigkeit mit einer vorbestimmten Flussrate transportiert und sichergestellt wird, dass in beiden Zeiträumen diese vorbestimmte Flussrate dieselbe ist. Diese zeitliche Versetzung der Flüsse durch Flussmesszelle K1 und Flussmesszelle K2 kann dabei besonders vorteilhaft in der Peritonealdialyse eingesetzt werden.

## Patentansprüche

1. Bilanziervorrichtung (201, 301, 303) zur Bestimmung einer Flüssigkeitsbilanz zwischen einer Flussmenge in einem einen Zufluss in eine Dialysierflüssigkeitskammer eines Dialysators bildenden ersten Flussweg (FW1) eines Dialysierflüssigkeitskreislaufs und einer Flussmenge in einem einen Abfluss aus der Dialysierflüssigkeitskammer des Dialysators bildenden zweiten Flussweg (FW2) des Dialysierflüssigkeitskreislaufs, enthaltend
eine Differenzflussmesseinheit (D) zum Messen des Differenzflusses zwischen einem Fluss in einer in dem Dialysierflüssigkeitskreislauf stromab der Dialysierflüssgkeitskammer angeordneten Flussmesszelle (K2) und einem Fluss in einer in dem Dialysierflüssigkeitskreislauf stromauf der Dialysierflüssigkeitskammer angeordneten Flussmesszelle (K1), wobei die erste Flussmesszelle (K1) im ersten Flussweg (FW1) und die zweite Flussmesszelle (K2) im zweiten Flussweg (FW2) angeordnet ist, wobei die Bilanziervorrichtung **gekennzeichnet ist durch** eine stromab der ersten Flussmesszelle (K1) angeordnete Verzweigung von dem ersten Flussweg zur Abzweigung von Flüssigkeit von dem ersten Flussweg (FW1) in einen zu der ersten Flussmesszelle parallelen weiteren Flussweg (W),
wobei der weitere Flussweg (W) stromauf der ersten Flussmesszelle wieder in den ersten Flussweg einmündet,
eine Einrichtung zur Einstellung der Flussmenge (P12, P14) in dem weiteren Flussweg, die derart ansteuerbar ist, dass der gemessene Differenzfluss eine vorbestimmte Bedingung erfüllt, und eine Einrichtung (K) zur Ermittlung der Flussmenge in dem weiteren Flussweg als Maß für die Menge des im Dialysator entzogenen Ultrafiltrats, und wobei die Einrichtung zur Einstellung der Flussmenge (P12, P14) in dem weiteren Flussweg als einstellbare Pumpe (P11, P12) ausgeführt ist, und wobei die vorbestimmte Bedingung erfüllt ist, wenn der Differenzfluss ungefähr null ist.

2. Bilanziervorrichtung (200) zur Bestimmung einer Flüssigkeitsbilanz zwischen einer Flussmenge in einem einen Zufluss in eine Dialysierflüssigkeitskammer eines Dialysators bildenden ersten Flussweg (FW1) eines Dialysierflüssigkeitskreislaufs und einer Flussmenge in einem einen Abfluss aus der Dialysierflüssigkeitskammer des Dialysators bildenden zweiten Flussweg (FW2) des Dialysierflüssigkeitskreislaufs, enthaltend
eine Differenzflussmesseinheit (D) zum Messen des Differenzflusses zwischen einem Fluss in einer in dem Dialysierflüssigkeitskreislauf stromab der Dialysierflüssigkeitskammer angeordneten Flussmesszelle (K2) und einem Fluss in einer in dem Dialysierflüssigkeitskreislauf stromauf der Dialysierflüssigkeitskammer angeordneten Flussmesszelle (K1), wobei die erste Flussmesszelle im ersten Flussweg (FW1) und die zweite Flussmesszelle im zweiten Flussweg (FW2) angeordnet ist,
wobei die Bilanziervorrichtung **gekennzeichnet ist durch**
eine stromauf der zweiten Flussmesszelle (K2) angeordnete Verzweigung von dem zweiten Flussweg zur Abzweigung von Flüssigkeit von dem zweiten Flussweg (FW2) in einen zu der zweiten Flussmesszelle parallelen weiteren Flussweg (W),
wobei der weitere Flussweg (W) stromab der zweiten Flussmesszelle wieder in den zweiten Flussweg einmündet,
eine Einrichtung zur Einstellung der Flussmenge (P1, P11) in dem weiteren Flussweg, die derart ansteuerbar ist, dass der gemessene Differenzfluss eine vorbestimmte Bedingung erfüllt,
und eine Einrichtung (K) zur Ermittlung der Flussmenge in dem weiteren Flussweg als Maß für die Menge des im Dialysator entzogenen Ultrafiltrats, und wobei die Einrichtung zur Einstellung der Flussmenge (P1, P11) in dem weiteren Flussweg als einstellbare Pumpe (P1, P11) ausgeführt ist, und wobei die vorbestimmte Bedingung erfüllt ist, wenn der Differenzfluss ungefähr null ist.

3. Bilanziervorrichtung (200, 201, 301, 303) nach einem der vorangehenden Ansprüche, wobei die Einrichtung zur Einstellung der Flussmenge (P1, P11, P12, P14) in dem weiteren Flussweg (W) die Einrichtung zur Ermittlung der Flussmenge in dem weiteren Flussweg (P1, P11, P12, P14) beinhaltet.

4. Bilanziervorrichtung (200, 201, 301, 303) nach einem der vorhergehenden Ansprüche, wobei die Differenzflussmesseinheit (D) als ein Differenzflusssensor zur unmittelbaren Messung des Differenzflusses zwischen dem Fluss im ersten Flussweg und dem Fluss im zweiten Flussweg ohne eine separate Messung des Flusses im ersten Flussweg oder eine separate Messung des Flusses im zweiten Flussweg ausgebildet ist.

5. Bilanziervorrichtung (200, 201, 301, 303) nach Anspruch 4, wobei der Differenzflusssensor eine erste Flusssmesszelle (K1) im ersten Flussweg (FW1) und eine zweite zur ersten Flussmesszelle gegenläufig durchströmte Flussmesszelle (K2) im zweiten Flussweg (FW2) aufweist.

6. Bilanziervorrichtung (200, 201, 301, 303) nach einem der vorangehenden Ansprüche wobei der erste und/oder der zweite Flusssensor eine Flussmesszelle ausgeführt als Volumenstromsensor oder Massenstromsensor ist.

7. Bilanziervorrichtung (200, 201, 301, 303) nach einem der vorhergehenden Ansprüche, wobei der weitere Flussweg ein absperrbares Ventil aufweist.

8. Bilanziervorrichtung (200, 201, 301, 303) nach einem der vorhergehenden Ansprüche, wobei der Differenzfluss als Flussrate oder als Flussvolumen bestimmt wird.

9. Bilanziervorrichtung (200, 201, 301, 303) nach einem der vorhergehenden Ansprüche, teilweise oder vollständig ausgeführt als Wegwerfartikel oder als Teil eines Wegwerfartikels.

10. Bilanziervorrichtung (200, 201, 301, 303) nach einem der vorhergehenden Ansprüche, wobei der Differenzfluss als Differenzvolumen, als Integral eines Differenzflusses, oder als Differenz eines Integrals des Flusses im ersten Flussweg und eines Integrals des Flusses im zweiten Flussweg messbar ist.

11. Dialysegerät umfassend eine Bilanziervorrichtung (200, 201, 301, 303) nach einem der Ansprüche 1 bis 10 zur Bilanzierung von Dialysierflüssigkeit.

12. Extrakorporale Blutbehandlungseinheit, mit einem Blutweg und einem Dialysierflüssigkeitsweg enthaltend eine Bilanziervorrichtung nach einem der Ansprüche 1 bis 10 zur Bilanzierung von Dialysierflüssigkeit in dem Dialysierflüssigkeitsweg.

13. Verfahren (500, 600) zur Bestimmung einer Flüssigkeitsbilanz zwischen einer Flussmenge in einem ersten Flussweg als Zufluss in eine Dialysierflüssigkeitskammer eines in einem Dialysierflüssigkeistskreislauf angeordneten Dialysators und einer Flussmenge in einem zweiten Flussweg als Abfluss aus der Dialysierflüssigkeitskammer des Dialysators, mit einer Bilanziervorrichtung nach einem der Ansprüche 1 bis 10, wobei das Verfahren die folgenden Schritte umfasst:
- Messen eines Differenzflusses zwischen einem Fluss im ersten Flussweg und einem Fluss im zweiten Flussweg (S1, S62),
- Verwenden des gemessenen Differenzflusses als Stellgröße für die Einrichtung zur Einstellung der Flussmenge im weiteren Flussweg (S2, S63) wobei die Einrichtung zur Einstellung der Flussmenge (P1, P11, P12, P14) in dem weiteren Flussweg als einstellbare Pumpe (P1, P11, P12, P14) ausgeführt ist und wobei die vorbestimmte Bedingung erfüllt ist, wenn der Differenzfluss ungefähr null ist und
- Ermitteln der Flussrate in dem weiteren Flussweg als Maß für die Menge des im Dialysator entzogenen Ultrafiltrats.

## Claims

1. A balancing device (201, 301, 303) for determining a fluid balance between a flow quantity in a first flow path (FW1) of a dialysis fluid circulation forming an inflow into a dialysis fluid chamber of a dialyzer and a flow quantity in a second flow path (FW2) of the dialysis fluid circulation forming an outflow out of the dialysis fluid chamber of the dialyzer, comprising
a differential flow-measuring unit (D) for measuring the differential flow between a flow in a flow-measuring cell (K2) disposed downstream from the dialysis fluid chamber in the dialysis fluid circulation,
and a flow in a flow-measuring cell (K1) disposed upstream from the dialysis fluid chamber in the dialysis fluid circulation,
wherein the first flow-measuring cell (K1) is disposed in the first flow path (FW1), and the second flow-measuring cell (K2) is disposed in the second flow path (FW2),
wherein the balancing device is **characterized by** a branch from the first flow path disposed downstream from the first flow-measuring cell (K1) for branching fluid off from the first flow path (FW1) into an additional flow path (W) parallel to the first flow-measuring cell,
wherein the additional flow path (W) discharges into the first flow path upstream from the first flow-measuring cell,
a device for adjusting the flow quantity (P12, P14) in the additional flow path, which can be controlled in such a way that the measured differential flow fulfills a predetermined condition,
and a device (K) for determining the flow quantity in the additional flow path as a measure of the amount of ultrafiltrate withdrawn in the dialyzer, and wherein the device for adjusting the flow quantity (P12, P14) in the additional flow path is embodied as an adjustable pump (P11, P12), and wherein the predetermined condition is satisfied when the differential flow is approximately zero.

2. A balancing device (200) for determining a fluid balance between a flow quantity in a first flow path (FW1) of a dialysis fluid circulation, forming an inflow into a dialysis fluid chamber of a dialyzer and a flow quantity in a second flow path (FW2) of the dialysis fluid circulation, forming an outflow out of the dialysis fluid chamber of the dialyzer, comprising
a differential flow-measuring unit (D) for measuring the differential flow between a flow in a flow-measuring cell (K2) disposed downstream from the dialysis fluid chamber in the dialysis fluid circulation
and a flow in a flow-measuring cell (K1) disposed upstream from the dialysis fluid chamber in the dialysis fluid circulation,
wherein the first flow-measuring cell (K1) is disposed in the first flow path (FW1), and the second flow-measuring cell (K2) is disposed in the second flow path (FW2),
wherein the balancing device is **characterized by** a branch from the second flow path disposed upstream from the second flow-measuring cell (K2) for diverting fluid from the second flow path (FW2) into an additional flow path (W) parallel to the second flow-measuring cell,
wherein the additional flow path (W) discharges into the second flow path downstream from the second flow-measuring cell,
a device for adjusting the flow quantity (P1, P11) in the additional flow path, which can be controlled in such a way that the measured differential flow fulfills a predetermined condition,
and a device (K) for determining the flow quantity in the additional flow path as a measure of the amount of ultrafiltrate withdrawn in the dialyzer, and wherein the device for adjusting the flow quantity (P1, P11) in the additional flow path
is embodied as an adjustable pump (P1, P11), and wherein the predetermined condition is met when the differential flow is approximately zero.

3. The balancing device (200, 201, 301, 303) according to any one of the preceding claims, wherein the device for adjusting the flow quantity (P1, P11, P12, P14) in the additional flow path (W) includes the device for determining the flow quantity in the additional flow path (P1, P11, P12, P14).

4. The balancing device (200, 201, 301, 303) according to any one of the preceding claims, wherein the differential flow-measuring unit (D) is designed as a differential flow sensor for direct measurement of the differential flow between the flow in the first flow path and the flow in the second flow path without a separate measurement of the flow in the first flow path or a separate measurement of the flow in the second flow path.

5. The balancing device (200, 201, 301, 303) according to claim 4, wherein the differential flow sensor comprises a first flow-measuring cell (K1) and the first flow path (FW1) and a second flow-measuring cell (K2) in the second flow path (FW2), through which the flow passes in the opposite direction from the first flow-measuring cell.

6. The balancing device (200, 201, 301, 303) according to any one of the preceding claims, wherein the first and/or second flow sensor(s) is/are a flow-measuring cell designed as a volume flow sensor or mass flow sensor.

7. The balancing device (200, 201, 301, 303) according to any one of the preceding claims, wherein the additional flow path has a valve that can be cut off.

8. The balancing device (200, 201, 301, 303) according to any one of the preceding claims, wherein the differential flow is determined as flow quantity or as flow volume.

9. The balancing device (200, 201, 301, 303) according to any one of the preceding claims, partially or completely embodied as a disposable article or as part of a disposable article.

10. The balancing device (200, 201, 301, 303) according to any one of the preceding claims, wherein the differential flow can be measured as a differential flow volume, as an integral of a differential flow or as the difference between an integral of the flow in the first flow path and an integral of the flow in the second flow path.

11. A dialysis machine, comprising a balancing device (200, 201, 301, 303) according to any one of claims 1 to 10 for balancing dialysis fluid.

12. An extracorporeal blood treatment unit, having a blood path and a dialysis flow path, comprising a balancing device according to any one of claims 1 to 10 for balancing dialysis fluid in the dialysis fluid path.

13. A method (500, 600) for determining a fluid balance between a flow quantity in a first flow path as the inflow into a dialysis fluid chamber of a dialyzer disposed in a dialysis fluid circulation and a flow quantity in a second flow path as the outflow out of the dialysis fluid chamber of the dialyzer, having a balancing device according to any one of claims 1 to 10, wherein the method comprises the following steps:
- measuring a differential flow between a flow in the first flow path and a flow in the second flow path (S1, S62),
- using the measured differential flow as a control variable for the device for adjusting the flow quantity in the additional flow path (S2, S63), wherein the device for adjusting the flow quantity (P1, P11, P12, P14) in the additional flow path is designed as an adjustable pump (P1, P11, P12, P14), and wherein the predetermined condition is satisfied when the differential flow is approximately zero, and
- determining the flow quantity in the additional flow path as a measure of the amount of ultrafiltrate withdrawn in the dialyzer.

## Revendications

1. Dispositif d'équilibrage (201, 301, 303) pour déterminer un bilan de liquide entre une quantité d'écoulement dans un premier parcours d'un circuit de liquide de dialyse (FW1) constituant un afflux dans une chambre de liquide de dialyse d'un dialyseur et une quantité d'écoulement dans un second parcours d'écoulement (FW2) du circuit de liquide de dialyse constituant une évacuation hors de la chambre de liquide de dialyse du dialyseur, contenant
une unité de mesure d'écoulement différentiel (D) pour mesurer l'écoulement différentiel entre un écoulement dans une cellule de mesure d'écoulement (K2) disposée dans le circuit de liquide de dialyse en aval de la chambre de liquide de dialyse et
un écoulement dans une cellule de mesure d'écoulement (Kl) disposée dans le circuit de liquide de dialyse en amont de la chambre de liquide de dialyse,
la première cellule de mesure d'écoulement (Kl) étant disposée dans le premier parcours d'écoulement (FW1) et la seconde cellule de mesure d'écoulement (K2) dans le second parcours d'écoulement (FW2),
le dispositif d'équilibrage étant **caractérisé par**
un embranchement disposé en aval de la première cellule de mesure (Kl) pour dévier du liquide du premier parcours d'écoulement (FW1) dans un autre parcours d'écoulement (W) parallèle à la première cellule de mesure d'écoulement,
l'autre parcours d'écoulement (W) débouchant à nouveau en amont de la première cellule de mesure d'écoulement dans le premier parcours d'écoulement,
un dispositif de réglage de la quantité d'écoulement (P12, P14) situé dans l'autre parcours d'écoulement et qui peut être commandé de manière à ce que l'écoulement différentiel mesuré remplisse une condition prédéterminée,
et un dispositif (K) de détermination de la quantité d'écoulement dans l'autre parcours d'écoulement servant d'indice pour la quantité d'ultrafiltrat extrait dans le dialyseur, le dispositif de réglage de la quantité d'écoulement (P12, P14) dans l'autre parcours d'écoulement étant réalisé sous forme d'une pompe réglable (P12, P14) et la condition prédéterminée étant remplie lorsque l'écoulement différentiel est approximativement nul.

2. Dispositif d'équilibrage (201, 301, 303) pour déterminer une bilan de liquide entre une quantité d'écoulement dans un premier parcours d'un circuit de liquide de dialyse constituant un afflux dans une chambre de liquide de dialyse d'un dialyseur et une quantité d'écoulement dans un second parcours d'écoulement (FW2) du circuit de liquid de dialyse constituant une évacuation hors de la chambre de liquide de dialyse du dialyseur, contenant
une unité de mesure d'écoulement différentiel (D) pour mesurer l'écoulement différentiel entre un écoulement dans une cellule de mesure d'écoulement disposée dans le circuit de liquide de dialyse en aval de la chambre de liquide de dialyse (K2) et
une cellule de mesure d'écoulement (K1) disposée dans le circuit de liquide de dialyse en amont de la chambre de liquide de dialyse,
la première cellule de mesure d'écoulement (K1) étant disposée dans le premier parcours d'écoulement (FW1) et la seconde cellule de mesure d'écoulement (K2) dans le second parcours d'écoulement (FW2),
le dispositif d'équilibrage étant **caractérisé par** un embranchement disposé en amont de la seconde cellule de mesure d'écoulement (K2) pour dévier du liquide du second parcours d'écoulement (F21) dans un autre parcours d'écoulement (W) parallèle à la seconde cellule de mesure d'écoulement,
l'autre parcours d'écoulement (W) débouchant de nouveau en aval de la seconde cellule de mesure d'écoulement dans le second parcours d'écoulement,
un dispositif de réglage de la quantité d'écoulement (P1, P11) dans l'autre parcours d'écoulement et qui peut être commandé de manière à ce que l'écoulement différentiel mesuré remplisse une condition prédéterminée,
et doté d'un dispositif (K) de détermination de la quantité d'écoulement dans l'autre parcours d'écoulement servant d'indice pour la quantité d'ultrafiltrat extrait dans le dialyseur, le dispositif de réglage de la quantité d'écoulement étant réalisé dans l'autre parcours d'écoulement sous forme d'une pompe réglable (P1, P11) et la condition prédéterminée étant remplie lorsque l'écoulement différentiel est approximativement nul.

3. Dispositif d'équilibrage (200, 201, 301, 303) selon une des revendications précédentes, dans lequel le dispositif de réglage de la quantité d'écoulement (P1, P11, P12, P14) dans l'autre parcours d'écoulement (W) contient le dispositif de détermination de la quantité d'écoulement dans l'autre parcours d'écoulement (P1, P11, P12, P14).

4. Dispositif d'équilibrage (200, 201, 301, 303) selon une des revendications précédentes, dans lequel l'unité de mesure d'écoulement différentiel (D) se présente sous la forme d'un capteur d'écoulement différentiel pour la mesure directe de l'écoulement différentiel entre l'écoulement dans le premier parcours d'écoulement et l'écoulement dans le second parcours d'écoulement sans mesure séparée de l'écoulement dans le premier parcours d'écoulement ou mesure séparée de l'écoulement dans le second parcours d'écoulement.

5. Dispositif d'équilibrage (200, 201, 301, 303) selon la revendication 4, dans lequel le capteur d'écoulement différentiel présente une première cellule de mesure d'écoulement (Kl) dans le premier parcours d'écoulement (FW1) et une seconde cellule de mesure d'écoulement (K2) parcourue par l'écoulement dans le sens inverse à la première cellule de mesure d'écoulement dans le second parcours d'écoulement (FW2).

6. Dispositif d'équilibrage (200, 201, 301, 303) selon une des revendications précédentes, dans lequel le premier et/ou le second capteur d'écoulement est une cellule de mesure d'écoulement réalisée sous forme d'un capteur de débit volumique ou d'un capteur de débit massique.

7. Dispositif d'équilibrage (200, 201, 301, 303) selon une des revendications précédentes, dans lequel l'autre parcours d'écoulement présente une soupape pouvant être bloquée.

8. Dispositif d'équilibrage (200, 201, 301, 303) selon une des revendications précédentes, dans lequel l'écoulement différentiel est défini sous forme d'une vitesse d'écoulement ou d'un volume d'écoulement.

9. Dispositif d'équilibrage (200, 201, 301, 303) selon une des revendications précédentes, réalisée partiellement ou complètement sous forme d'un article jetable ou en tant qu'élément d'un article jetable.

10. Dispositif d'équilibrage (200, 201, 301, 303) selon une des revendications précédentes, dans lequel l'écoulement différentiel est mesurable sous forme de volume différentiel, d'intégrale d'un écoulement différentiel ou sous forme de différence entre une intégrale de l'écoulement dans le premier parcours d'écoulement et d'une intégrale de l'écoulement dans le second parcours d'écoulement.

11. Appareil de dialyse comprenant un dispositif d'équilibrage (200, 201, 301, 303) selon une des revendications 1 à 10 pour l'équilibrage de liquide de dialyse.

12. Unité extracorporelle de traitement sanguin, comportant un parcours de sanguin et un parcours de liquide de dialyse, contenant un dispositif d'équilibrage selon une des revendications 1 à 10 pour l'équilibrage de liquide de dialyse dans le parcours de liquide de dialyse.

13. Procédé (500, 600) de déterminer un bilan de liquide entre une quantité d'écoulement dans un premier parcours d'écoulement faisant office d'afflux dans une chambre de liquide de dialyse d'un dialyseur disposé dans un circuit de liquide de dialyse et une quantité d'écoulement dans un second parcours d'écoulement faisant office d'évacuation hors de la chambre de liquide de dialyse du dialyseur, comportant un dispositif d'équilibrage selon une des revendications 1 à 10, ce procédé comprenant les étapes suivantes
- mesure d'un écoulement différentiel entre un écoulement dans le premier parcours d'écoulement et un écoulement dans le second parcours d'écoulement (S1, S62),
- utilisation de l'écoulement différentiel mesuré comme paramètre de réglage pour le dispositif de réglage de la quantité d'écoulement dans l'autre parcours d'écoulement (S2, S63), le dispositif de réglage de la quantité d'écoulement (P1, P11, P12, P14) étant réalisé dans l'autre parcours d'écoulement sous forme d'une pompe réglable (P1, P11, P12, P14) et la condition prédéterminée étant remplie lorsque l'écoulement différentiel est approximativement nul et
- détermination de la vitesse d'écoulement dans l'autre parcours d'écoulement en tant qu'indice de la quantité d'ultrafiltrat extraite dans le dialyseur.
